# EUROPEAN PATENT APPLICATION

(11) **EP 4 458 376 A1**
(43) Date of publication of application: **06.11.2024**
(21) Application number: 23746143.9
(22) Date of filing: 17.01.2023
(51) Int. Cl.: A61K 45/00, A61K 47/24, A61K 47/12, A61K 9/127, A61K 31/485, A61K 31/4468, A61K 31/4535, A61K 31/454, A61P 25/04, A61P 25/02, A61P 29/00

(54) **PH-RESPONSIVE ANALGESIC AGENT FOR TARGETING DAMAGED SEGMENT DORSAL ROOT GANGLION AND USE THEREOF**

(30) Priority: 30.01.2022 CN 202210113469
(71) Applicant: Zhejiang University, Hangzhou, Zhejiang 310058 (CN)
(72) Inventor: TANG, Jianbin, Hangzhou, Zhejiang 310027 (CN); YAN, Min, Hangzhou, Zhejiang 310027 (CN); MA, Longfei, Hangzhou, Zhejiang 310027 (CN); YUAN, Pengcheng, Hangzhou, Zhejiang 310027 (CN); YU, Lina, Hangzhou, Zhejiang 310027 (CN)
(74) Representative: karo IP
(86) International application number: PCT/CN2023/072651
(87) International publication number: WO 2023/143261

(57) **Abstract**

The present invention discloses a pH-responsive analgesic drug targeting an injured segment of a dorsal root ganglion and use thereof and belongs to the technical field of biomedicine. On the basis that the injured segment of the dorsal root ganglion continuously exists in a local acidic environment under the condition of neuropathic pain caused by a peripheral nerve injury, the present invention proposes to prepare the analgesic drug for the peripheral nerve injury by using the injured segment of the dorsal root ganglion as a drug action target, and embedding the analgesic drug in a pH-responsive carrier, such that the analgesic drug is released at the site of the injured segment of the dorsal root ganglion and then plays a role to achieve an analgesic effect reaching an analgesic level of systemic action of the same drug. The present invention realizes precise medication at an acidic focus to remarkably reduce the release of the drug at other sites and greatly reduce toxic and side effects such as addiction and the like in medication of existing opioids. In addition, the drug is continuously and slowly released at the injured segment of the dorsal root ganglion to remarkably prolong an analgesic action time and avoid excessive use of the analgesic drug.

## Description

### FIELD OF TECHNOLOGY

The present invention relates to the technical field of biomedicine and specifically relates to an analgesic drug targeting an injured segment of a dorsal root ganglion and the use thereof in the preparation of drugs for the treatment of neuropathic pain caused by a peripheral nerve injury.

### BACKGROUND

Opioids, namely narcotic analgesic drugs, are the most commonly used drugs for the treatment of moderate to severe acute and chronic pain. The opioids exert an analgesic effect by binding to opioid receptors of peripheral and central nervous systems (spinal cord and brain). At present, the opioid receptors that have been confirmed include four types, including µ, κ, δ, and orphanin, in which the µ, κ, and δ receptors are all associated with analgesia.

Opioids have a variety of types and can be divided into strong opioids and weak opioids according to differences in analgesic intensity. During the treatment of cancer pain, according to a three-step treatment principle of the World Health Organization (WHO), weak opioids are two-step drugs, and strong opioids are three-step drugs. The weak opioids include codeine, dihydrocodeine, etc., which are mainly used for analgesia of mild to moderate acute pain; and the strong opioids include morphine, fentanyl, meperidine, sufentanyl, oxycodone and hydromorphone, etc., which are mainly used for treatment of moderate to severe postoperative pain.

Highly effective pure agonist-type opioids have a strong analgesic effect, no organ toxicity, and no ceiling effect, and are under the principle of maximum analgesia without generating unbearable adverse reactions when used. However, the analgesic effect and adverse reactions of the opioids are both dose-dependent and acceptor-dependent, and side effects are mainly shown as respiratory depression, addiction, drug resistance, physiological dependence, difficult withdrawal, muscle rigidity, myoclonus, convulsion, constipation, etc. Clinically, opioids are an indispensable part in treatment of chronic pain, and as highly effective analgesic drugs, have a good analgesic effect. However, opioids have many serious side effects due to long-term use, such that long-term use of opioids in chronic pain is limited.

How to use opioids that can continuously relieve pain without causing damage (such as abuse, excessive use, and addiction) is a challenging problem.

At present, in targeted treatment of neuropathic pain, it has been reported that liposome-encapsulated morphine or oxymorphone can significantly prolong an analgesic time after subcutaneous injection, but due to the lack of targeting specificity, cannot accurately act on a lesion site (Smith LJ, et al. A single dose of liposome-encapsulated hydromorphone provides extended analgesia in a rat model of neuropathic pain. Comp Med. 2006 Dec; 56(6):487-92.), (Smith LJ, et al. A single dose of liposome-encapsulated oxymorphone or morphine provides long-term analgesia in an animal model of neuropathic pain. Comp Med. 2003 Jun; 53(3):280-7.). It has also been reported that using characteristics of an acidic environment in an endosome, an acid-responsive polymer containing a neurokinin is designed, which can simultaneously act on central and peripheral endosomes to relieve pain. However, the polymer has too extensive action sites, thus inevitably causing a series of side effects. Meanwhile, a complex polymer structure also increases the clinical transformation difficulty (Ramirez-Garcia PD, et al. A pH-responsive nanoparticle targets the neurokinin 1 receptor in endosomes to prevent chronic pain. Nat Nanotechnol. 2019 Dec; 14(12): 1150-1159.).

Therefore, the development of an analgesic drug having targetability to reduce the generation of side effects is a problem to be solved urgently by persons skilled in the art.

### SUMMARY

The present invention aims to overcome the above disadvantages and provide an analgesic drug that can not only achieve effective analgesia but also solve the problem of a series of side effects of opioids.

In order to achieve the above purpose, the following technical schemes are adopted in the present invention.

The present invention provides the use of an injured segment of a dorsal root ganglion as a target in preparation of an analgesic drug for a peripheral nerve injury, where the analgesic drug is a pH-responsive analgesic drug targeting the injured segment of the dorsal root ganglion.

As found through previous studies of the present invention, only the injured segment of the dorsal root ganglion (DRG) continuously exists in a local acidic environment under the condition of neuropathic pain caused by the peripheral nerve injury, and other segments of the dorsal root ganglion do not have the phenomenon. Effective analgesia can be achieved by activating a µ opioid receptor at the site of the injured segment of the dorsal root ganglion.

Based on such discovery, the present invention proposes to prepare a pH-responsive analgesic drug targeting an injured segment of a dorsal root ganglion, which takes the injured segment of the dorsal root ganglion as a drug action target to achieve treatment of pain caused by a peripheral nerve injury.

The pH-responsive analgesic drug means that an active analgesic ingredient is released to exert an analgesic effect only under the condition of an acidic environment (pH<7).

The peripheral nerve injury refers to the injury of nerves except for a central nervous system (brain and spinal cord), including 12 pairs of cranial nerves and 31 pairs of spinal nerves and autonomic nerves.

Further, the pH-responsive analgesic drug is a complex formed by embedding the active analgesic ingredient in a pH-responsive carrier, that is, the pH-responsive analgesic drug is composed of the pH-responsive carrier and the analgesic drug embedded in the carrier.

As a preference, the pH-responsive carrier is a pH-responsive liposome. In the present invention, a drug dosage form is changed by embedding the analgesic drug in the pH-responsive liposome, which can greatly reduce the drug entering the central nervous system through a blood-brain barrier and reduce the toxic and side effects of the analgesic drug. Meanwhile, the drug can target the injured segment of the dorsal root ganglion (DRG) and release the active ingredient of the analgesic drug using an acidic environment at an acidic focus to exert the analgesic effect.

The pH-responsive liposome may be composed of a pH-responsive lipid molecule and may also be composed of other pH-responsive polymers.

Specifically, a hydrophilic portion of the pH-responsive liposome includes pH-responsive groups, and these groups include a deprotonable carboxyl group or a protonable amino group.

As a preference, the composition of the pH-responsive liposome includes phosphatidylethanolamine and oleic acid. By adjusting the ratio of the two lipids, the pH responsiveness can be changed, such that the drug is effectively released at an acidic pH value of the injured dorsal root ganglion.

Further, the active ingredient of the pH-responsive analgesic drug is an opioid. Studies show that the dorsal root ganglion has an opioid receptor, and the opioid drug is bound to the opioid receptor to achieve the analgesic effect. Specifically, the opioid is a µ opioid receptor agonist, including but not limited to morphine, fentanyl, remifentanyl, afentanyl, hydromorphone, sulfentanyl, nalbuphine hydrochloride, butorphanol, and oxycodone.

Further, the present invention provides an analgesic drug targeting an injured segment of a dorsal root ganglion, where the analgesic drug includes a pH-responsive carrier and an opioid embedded in the carrier. The pH-responsive analgesic drug is prepared by embedding the opioid in the pH-responsive carrier.

As a preference, the pH-responsive carrier is a pH-responsive liposome and has a composition including phosphatidylethanolamine and oleic acid.

The present invention provides a preparation method for the pH-responsive analgesic drug embedded in the opioid, including the following steps:
(1) dissolving the phosphatidylethanolamine and the oleic acid in an organic solvent, and removing the organic solvent under reduced pressure to form a lipid film; and
(2) adding a buffer solution containing the opioid to undergo a hydration reaction, and performing extrusion molding through a filter membrane after complete hydration to prepare the pH-responsive analgesic drug.

As a preference, the organic solvent is a low-boiling-point solvent, such as trichloromethane, ethyl ether, tetrahydrofuran, chloroform, etc. Through combination with ultrasonic treatment, raw materials are completely dissolved.

As a preference, in step (1), the molar ratio of the phosphatidylethanolamine to the oleic acid is (1.5-4):1. More preferably, the molar ratio of the phosphatidylethanolamine to the oleic acid is 7:3.

As a preference, after the phosphatidylethanolamine and the oleic acid are completely dissolved in the organic solvent, the pressure is slowly reduced from 400 MPa to 20 MPa at 35-55°C (until no bubbles are obviously generated on a liquid surface), a mixed solution is evaporated to dryness at a rotation speed of 30-120 r/min, and then the organic solvent is completely removed by vacuum drying.

In step (2), the opioid is added according to a mass ratio of the opioid to a film-forming material, which is controlled within 10%.

As a preference, the opioid is dissolved in a phosphate buffer solution to prepare an opioid buffer solution.

As a preference, conditions for the hydration reaction include that the hydration is performed at 35-55°C for 10-20 h. More preferably, the hydration is performed at 35-40°C for 12 h.

The particle size of the drug can be controlled by different filter membrane pore sizes, and considering use in organisms and decreased accumulation in the central nervous system such as the brain, a particle size range of a product is controlled in 50-400 nm. As a preference, in step (2), a hydration product is subjected to extrusion molding through a water phase filter membrane with a pore size of 220 nm.

The film-forming material used in the preparation method is simple and easy to obtain and has a huge price advantage, and a preparation process is simple. The pH-responsive analgesic drug prepared under the above conditions has a high encapsulation rate and stable performance. Studies show that the pH-responsive analgesic drug prepared by the present invention can not only continuously release the opioid at the injured segment of the dorsal root ganglion (DRG) to prolong the action time, but also remarkably reduce the amount of the drug entering the brain to reduce addiction and reduce side effects.

Further, the present invention provides the use of the analgesic drug targeting an injured segment of a dorsal root ganglion in preparation of drugs for treatment of pain caused by a peripheral nerve injury.

The pH-responsive analgesic drug releases the opioid active substance in a local acidic environment of the injured segment of the dorsal root ganglion to exert the analgesic effect.

In practical use, the drug can be prepared into an injection, a freeze-dried powder, or a spray for use.

The present invention has the following beneficial effects.
(1) On the basis that the injured segment of the dorsal root ganglion continuously exists in a local acidic environment under the condition of neuropathic pain caused by a peripheral nerve injury, the present invention proposes to prepare the analgesic drug for the peripheral nerve injury by using the injured segment of the dorsal root ganglion as a drug action target for the first time and embedding the analgesic drug in a pH-responsive carrier, such that the analgesic drug is released at the site of the injured segment of the dorsal root ganglion and then plays a role to achieve an analgesic effect reaching an analgesic level of systemic action of the same drug.
(2) Because the present invention realizes precise medication at an acidic focus, the release of the drug at other sites is remarkably reduced, and toxic and side effects such as addiction and the like in medication of existing opioids are greatly reduced.
(3) Through drug modification in the present invention, the drug is continuously and slowly released at the injured segment of the dorsal root ganglion to remarkably prolong an analgesic action time and avoid excessive use of the analgesic drug.

### BRIEF DESCRIPTION OF THE DRAWINGS

FIG. 1 shows dynamic light scattering data of pH-responsive analgesic drugs prepared in Examples 1-9.
FIG. 2 shows electron microscopic images of a pH-responsive morphine analgesic drug prepared in Example 1, where a scale of FIG. (A) is 100 nm, and a scale of FIG. (B) is 20 nm.
FIG. 3 shows a potential diagram of the pH-responsive morphine analgesic drug prepared in Example 1.
FIG. 4 shows morphine release curves of the pH-responsive morphine analgesic drug prepared in Example 1 under different pH conditions.

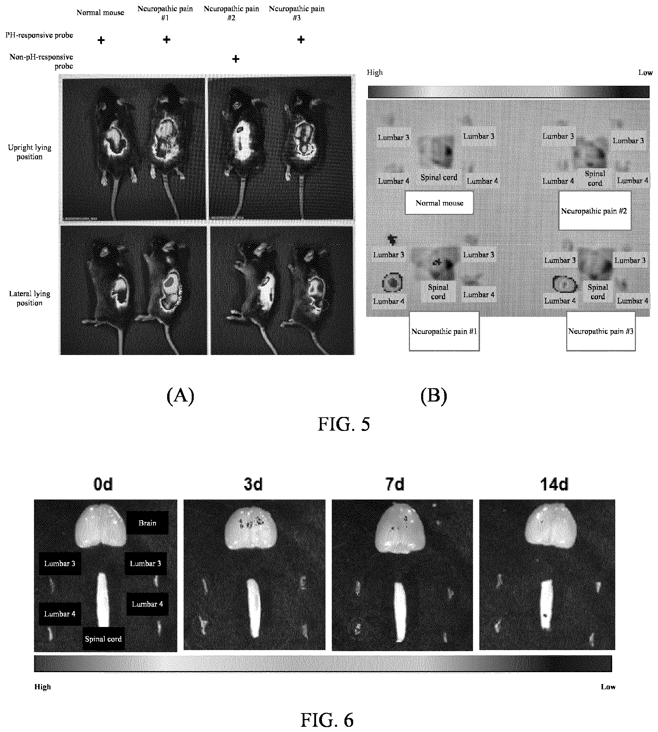

FIG. 5 shows the detection results of an acidic focus of a dorsal root ganglion of a neuropathic pain model, where FIG. (A) shows systemic distribution conditions of fluorescence signals 6 h after mice are administered with acid-responsive or non-acid-responsive small molecule probes through tail intravenous injection; and FIG. (B) shows fluorescence signal intensity of various tissues observed after ipsilateral lumbar 3 and 4 ganglia, contralateral lumbar 3 and 4 ganglia, and lumbar 3-4 segments of spinal cords of the living imaging mice are removed, where #1 is the 1st neuropathic pain model mouse, #2 is the 2nd neuropathic pain model mouse, and #3 is the 3rd neuropathic pain model mouse.
FIG. 6 shows the distribution conditions of a pH-responsive liposome in the brain, spinal cord, and various ganglia of mice 24 h after tail intravenous injection in a neuropathic pain model on day 0, day 3, day 7, and day 14.
FIG. 7 shows the amount of morphine in various tissues determined by HPLC when various ganglia are taken out 6 h after tail intravenous injection of the pH-responsive morphine analgesic drug prepared in Example 1 at a morphine dose of 5 mg kg⁻¹.
FIG. 8 shows the mechanical foot withdrawal frequency of mice observed after tail intravenous injection of the pH-responsive morphine analgesic drug prepared in Example 1 at a morphine dose of 5 mg kg⁻¹.
FIG. 9 shows the mechanical foot withdrawal frequency of mice observed after subcutaneous injection of the pH-responsive morphine analgesic drug prepared in Example 1 at a morphine dose of 10 mg kg⁻¹.
FIG. 10 shows the side effects observed after subcutaneous injection of the pH-responsive morphine analgesic drug prepared in Example 1 at 10 mg kg⁻¹, where (A) shows withdrawal symptoms, (B) shows the heart rate, (C) shows blood oxygen, (D) shows motor coordination, (E) shows movement distance, (F) shows typical movement trajectory diagrams, and (G) shows defecation conditions.

### DESCRIPTION OF THE EMBODIMENTS

The present invention is further described below in combination with specific embodiments. The following embodiments are used only to illustrate the present invention, rather than to limit the scope of application of the present invention. All modifications or substitutions of the method, steps, or conditions of the present invention that are made without departing from the spirit and essence of the present invention fall within the scope of the present invention.

All test methods used in the following embodiments, unless otherwise specified, are conventional methods; and all materials, reagents and the like used, unless otherwise specified, are commercially available reagents and materials.

Phosphatidylethanolamine has a CAS No. 39382-08-6; oleic acid has a CAS No. 112-80-1; morphine was purchased from Northeast Pharmaceutical Group First Pharmaceutical Co., Ltd.; sulfentanyl, remifentanyl, fentanyl, hydromorphone and afentanyl were purchased from Yichang Humanwell Pharmaceutical Co., Ltd.; a butorphanol injection was purchased from Jiangsu Hengrui Pharmaceutical Co., Ltd.; and an oxycodone hydrochloride injection (oxynorm) was purchased from RAFA LABORATORIES LIMITED.

### Example 1: Preparation of a pH-responsive morphine analgesic drug

0.1 g of phosphatidylethanolamine and 0.016 g of oleic acid were accurately weighed (at a molar ratio of 7/3) and placed in a 25 mL eggplant-shaped flask, and 6 mL of trichloromethane was added. After ultrasonic treatment was performed for 10 s for complete dissolution, the pressure (400-20 Mpa) was slowly reduced in a water bath pot at 35°C until no bubbles were obviously generated on a liquid surface, and a mixed solution was evaporated to dryness at a rotation speed of 90 r/min to form a layer of a uniform lipid film. An organic solvent was completely removed by vacuum drying for 24 h, then 15 mL of a morphine PBS buffer solution with a concentration of 0.133 mg mL⁻1 was added, a magneton was added for hydration under water bath conditions at 45°C for 12 h and extrusion molding was performed through a 220 nm aqueous phase filter membrane after complete hydration to obtain a pH-responsive morphine analgesic drug.

### Example 2: Preparation of a pH-responsive remifentanyl analgesic drug

0.1 mg of phosphatidylethanolamine and 0.016 mg of oleic acid were accurately weighed (at a molar ratio of 7/3) and placed in a 5 mL eggplant-shaped flask, and 2 mL of trichloromethane was added. After ultrasonic treatment was performed for 5 s for complete dissolution, the pressure (400-20 Mpa) was slowly reduced in a water bath pot at 40°C, and a mixed solution was evaporated to dryness at a rotation speed of 80 r/min to form a layer of a uniform lipid film. An organic solvent was completely removed by vacuum drying for 24 h, then 3 mL of a remifentanyl PBS buffer solution with a concentration of 100 µg mL⁻¹ was added, a magneton was added for hydration under water bath conditions at 35°C for 12 h, and extrusion molding was performed through a 220 nm aqueous phase filter membrane after complete hydration to obtain a pH-responsive remifentanyl analgesic drug.

### Example 3: Preparation of a pH-responsive afentanyl analgesic drug

0.86 mg of phosphatidylethanolamine and 0.21 mg of oleic acid were accurately weighed (at a molar ratio of 6/4) and placed in a 5 mL eggplant-shaped flask, and 2 mL of trichloromethane was added. After ultrasonic treatment was performed for 25 s for complete dissolution, the pressure (400-20 Mpa) was slowly reduced in a water bath pot at 40°C, and a mixed solution was evaporated to dryness at a rotation speed of 80 r/min to form a layer of a uniform lipid film. An organic solvent was completely removed by vacuum drying for 24 h, then 3 mL of an afentanyl PBS buffer solution with a concentration of 1.5 mg mL⁻¹ was added, a magneton was added for hydration under water bath conditions at 35°C for 12 h, and extrusion molding was performed through a 220 nm aqueous phase filter membrane after complete hydration to obtain a pH-responsive afentanyl analgesic drug.

### Example 4: Preparation of a pH-responsive hydromorphone analgesic drug

0.1 mg of phosphatidylethanolamine and 0.016 mg of oleic acid were accurately weighed (at a molar ratio of 7/3) and placed in a 25 mL eggplant-shaped flask, and 10 mL of ethyl ether was added. After ultrasonic treatment was performed for 50 s for complete dissolution, the pressure (400-20 Mpa) was slowly reduced in a water bath pot at 35°C, and a mixed solution was evaporated to dryness at a rotation speed of 90 r/min to form a layer of a uniform lipid film. An organic solvent was completely removed by vacuum drying for 24 h, then 15 mL of a hydromorphone PBS buffer solution with a concentration of 2 mg mL⁻¹ was added, a magneton was added for hydration under water bath conditions at 35°C for 12 h, and extrusion molding was performed through a 220 nm aqueous phase filter membrane after complete hydration to obtain a pH-responsive hydromorphone analgesic drug.

### Example 5: Preparation of a pH-responsive sulfentanyl analgesic drug

0.1 mg of phosphatidylethanolamine and 0.016 mg of oleic acid were accurately weighed (at a molar ratio of 7/3) and placed in a 25 mL eggplant-shaped flask, and 10 mL of trichloromethane was added. After ultrasonic treatment was performed for 10 s for complete dissolution, the pressure (400-20 Mpa) was slowly reduced in a water bath pot at 35°C, and a mixed solution was evaporated to dryness at a rotation speed of 90 r/min to form a layer of a uniform lipid film. An organic solvent was completely removed by vacuum drying for 24 h, then 15 mL of a sulfentanyl PBS buffer solution with a concentration of 50 µg mL⁻¹ was added, a magneton was added for hydration under water bath conditions at 35°C for 12 h, and extrusion molding was performed through a 220 nm aqueous phase filter membrane after complete hydration to obtain a pH-responsive sulfentanyl analgesic drug.

### Example 6: Preparation of a pH-responsive fentanyl analgesic drug

0.1 g of phosphatidylethanolamine and 0.016 g of oleic acid were accurately weighed (at a molar ratio of 7/3) and placed in a 25 mL eggplant-shaped flask, and 6 mL of trichloromethane was added. After ultrasonic treatment was performed for 10 s for complete dissolution, the pressure (400-20 Mpa) was slowly reduced in a water bath pot at 35°C, and a mixed solution was evaporated to dryness at a rotation speed of 70 r/min to form a layer of a uniform lipid film. An organic solvent was completely removed by vacuum drying for 24 h, then 5 mL of a fentanyl PBS buffer solution with a concentration of 50 µg mL⁻¹ was added, a magneton was added for hydration under water bath conditions at 35°C for 12 h, and extrusion molding was performed through a 220 nm aqueous phase filter membrane after complete hydration to obtain a pH-responsive fentanyl analgesic drug.

### Example 7: Preparation of a pH-responsive buprenorphine hydrochloride analgesic drug

0.1 g of phosphatidylethanolamine and 0.016 g of oleic acid were accurately weighed (at a molar ratio of 7/3) and placed in a 25 mL eggplant-shaped flask, and 6 mL of trichloromethane was added. After ultrasonic treatment was performed for 10 s for complete dissolution, the pressure (400-20 Mpa) was slowly reduced in a water bath pot at 35°C, and a mixed solution was evaporated to dryness at a rotation speed of 90 r/min to form a layer of a uniform lipid film. An organic solvent was completely removed by vacuum drying for 24 h, then 5 mL of a buprenorphine hydrochloride PBS buffer solution with a concentration of 10 mg mL⁻¹ was added, a magneton was added for hydration under water bath conditions at 35°C for 12 h, and extrusion molding was performed through a 220 nm aqueous phase filter membrane after complete hydration to obtain a pH-responsive buprenorphine hydrochloride analgesic drug.

### Example 8: Preparation of a pH-responsive butorphanol analgesic drug

0.01 g of phosphatidylethanolamine and 0.0016 g of oleic acid were accurately weighed (at a molar ratio of 7/3) and placed in a 10 mL eggplant-shaped flask, and 3 mL of trichloromethane was added. After ultrasonic treatment was performed for 10 s for complete dissolution, the pressure (400-20 Mpa) was slowly reduced in a water bath pot at 35°C, and a mixed solution was evaporated to dryness at a rotation speed of 90 r/min to form a layer of a uniform lipid film. An organic solvent was completely removed by vacuum drying for 24 h, then 3 mL of a butorphanol PBS buffer solution with a concentration of 1 mg mL⁻¹ was added, a magneton was added for hydration under water bath conditions at 35°C for 12 h, and extrusion molding was performed through a 220 nm aqueous phase filter membrane after complete hydration to obtain a pH-responsive butorphanol analgesic drug.

### Example 9: Preparation of a pH-responsive oxycodone analgesic drug

0.1 g of phosphatidylethanolamine and 0.016 g of oleic acid were accurately weighed (at a molar ratio of 7/3) and placed in a 25 mL eggplant-shaped flask, and 6 mL of trichloromethane was added. After ultrasonic treatment was performed for 10 s for complete dissolution, the pressure (400-20 Mpa) was slowly reduced in a water bath pot at 35°C, and a mixed solution was evaporated to dryness at a rotation speed of 80 r/min to form a layer of a uniform lipid film. An organic solvent was completely removed by vacuum drying for 24 h, then 5 mL of an oxycodone PBS buffer solution with a concentration of 5 mg mL⁻¹ was added, a magneton was added for hydration under water bath conditions at 35°C for 12 h, and extrusion molding was performed through a 220 nm aqueous phase filter membrane after complete hydration to obtain a pH-responsive oxycodone analgesic drug.

### Performance test examples

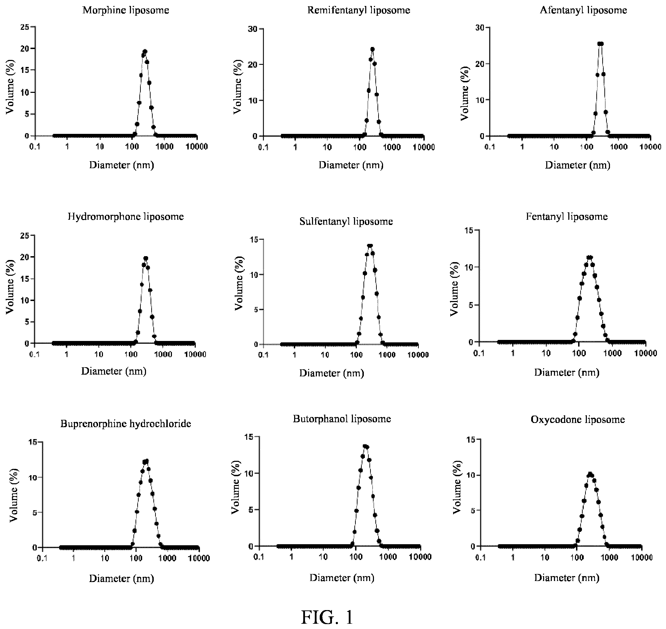

1. Particle sizes of the pH-responsive analgesic drugs prepared in Examples 1-9 were measured by dynamic light scattering. Results are shown in FIG. 1.
   The pH-responsive morphine analgesic drug prepared in Example 1 has an average particle size of 260.5 nm and a PDI value of 0.026 and has good uniformity.
   The pH-responsive remifentanyl analgesic drug prepared in Example 2 has a size of 259.8 nm and a PDI value of 0.061.
   The pH-responsive afentanyl analgesic drug prepared in Example 3 has a size of 281.0 nm and a PDI value of 0.327.
   The pH-responsive hydromorphone analgesic drug prepared in Example 4 has a size of 300.8 nm and a PDI value of 0.041.
   The pH-responsive sulfentanyl analgesic drug prepared in Example 5 has a size of 302.1 nm and a PDI value of 0.151.
   The pH-responsive fentanyl analgesic drug prepared in Example 6 has a size of 259.1 nm and a PDI value of 0.148.
   The pH-responsive buprenorphine hydrochloride analgesic drug prepared in Example 7 has a size of 219.1 nm and a PDI value of 0.163.
   The pH-responsive butorphanol analgesic drug prepared in Example 8 has a size of 233.6 nm and a PDI value of 0.169.
   The pH-responsive oxycodone analgesic drug prepared in Example 9 has a size of 221.3 nm and a PDI value of 0.200.

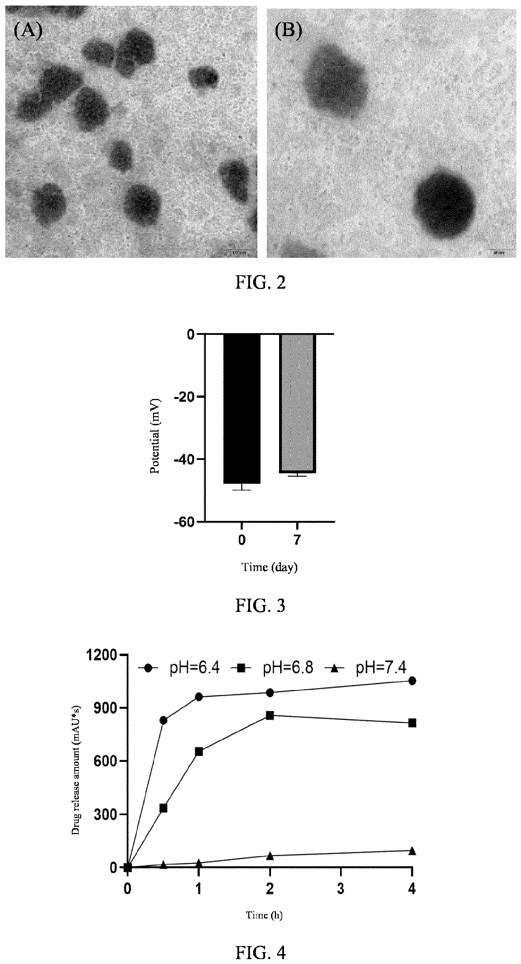

2. FIG. 2 shows TEM images of the pH-responsive morphine analgesic drug prepared in Example 1. Relatively regular spherical particles with good dispersion and a uniform size can be observed.
3. FIG. 3 shows a potential diagram of the pH-responsive morphine analgesic drug prepared in Example 1. The Zeta potential is measured to be -43.6 mV to -49.9 mV. Moreover, almost no great changes are found in a week, indicating that the analgesic drug has high stability.
4. Release conditions of the pH-responsive morphine analgesic drug prepared in Example 1 in PBS buffer solutions with different pH values were measured by a dynamic membrane dialysis method. Results are shown in FIG. 4. The pH-responsive analgesic drug has an encapsulation rate of 60%, the liposome drug has obvious differences in the release rate at different pH values, and the release rate under acidic conditions is significantly higher.

### Application examples

### 1. Construction of a neuropathic pain model caused by a peripheral nerve injury

Mouse lumbar 4 spinal nerve ligation model: First, mice were anesthetized, the skin of lower back portions of the mice was cut until lumbar transverse processes were exposed, lower segments of lumbar 4 spinal nerves were ligated with 6-0 sterile silk threads after the transverse processes were removed, and the spinal nerves were cut at ligated distal ends. Muscles and the skin were sutured layer by layer after surgery.

### 2. Detection of an acidic focus of a dorsal root ganglion

Mice in a model group were administered with acid-responsive or non-acid-responsive small molecule probes through tail intravenous injection, mice in a sham-operated group were treated the same, and systemic distribution of fluorescence signals was observed by a small animal living imaging instrument 6 h later.

Results are shown in FIG. 5. Only the mice administered with the acid-responsive small molecule probes through tail intravenous injection in the model group have strong signals appearing at the site of an injured side of a dorsal root ganglion (FIG. 5A); and by observing the fluorescence signal intensity of various tissues after ipsilateral lumbar 3 and lumbar 4 ganglia, contralateral lumbar 3 and lumbar 4 ganglia and lumbar 3-4 segments of spinal cords of the above living imaging mice are removed, it is found that the signal intensity of the ipsilateral lumbar 4 ganglion is significantly higher than that of the contralateral lumbar 4 ganglion and the ipsilateral lumbar 3 ganglia (FIG. 5B). The above results indicate that an injured side segment of the dorsal root ganglion has characteristics of a local acidic environment.

### 3. Detection of a target site of a pH-responsive liposome

With reference to a preparation method for a morphine liposome in Example 1, fluorescent molecules were added in a film-preparing process, and an appropriate amount of PBS was added for hydration after film formation to prepare a fluorescence-labeled liposome. Mice in a model group were administered with a pH-responsive liposome through tail intravenous injection on day 0, day 3, day 7, and day 14 after molding, respectively, and distribution of the liposome in the brain, spinal cord, and various ganglia was measured 24 h later.

As shown in FIG. 6, the fluorescence labeled liposome mainly appears at the site of an injured segment of a dorsal root ganglion (DRG), indicating that the pH-responsive liposome can effectively reduce the amount entering the brain while being released at the site of the DRG.

### 4. Metabolic release conditions of a pH-responsive analgesic drug

The pH-responsive morphine analgesic drug prepared in Example 1 was selected. After tail intravenous injection at a morphine dose of 5 mg kg⁻¹, various ganglia were taken out, and the amount of morphine in various tissues was determined by HPLC.

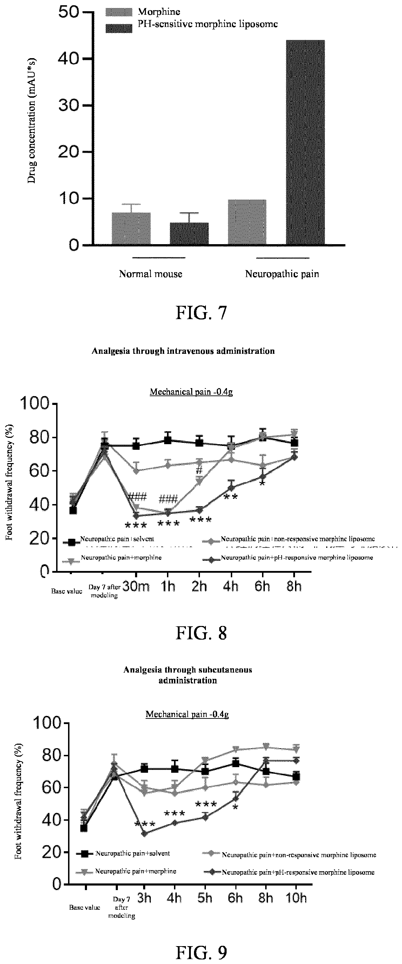

Results are shown in FIG. 7. The pH-responsive morphine analgesic drug is released at an injured DRG, and a concentration difference can reach 4 times compared with that of a morphine single drug. It is indicated that the morphine drug embedded in the pH-responsive liposome can effectively reduce release at other sites and reduce toxic and side effects while bringing a good therapeutic effect.

### 5. An analgesic effect of a pH-responsive analgesic drug

The pH-responsive morphine analgesic drug prepared in Example 1 was selected. After tail intravenous injection at a morphine dose of 5 mg kg⁻1 or subcutaneous injection at 10 mg kg⁻¹, an analgesic effect of the drug was reflected by observing the mechanical foot withdrawal frequency of mice.

Meanwhile, control groups were set, including mice with neuropathic pain that were administered with a drug solvent (PBS) through tail intravenous injection or subcutaneous injection, a morphine single drug (through tail intravenous injection at 5 mg kg⁻¹ or subcutaneous injection at 10 mg kg⁻¹), or a non-responsive morphine liposome (morphine liposome without pH respond, through tail intravenous injection at 5 mg kg⁻¹ or subcutaneous injection at 10 mg kg⁻¹).

Detection of mechanical foot withdrawal frequency: The skin in the center of the hind planta of mice was vertically stimulated by 0.4 g of a von Frey fiber filament through a metal mesh until the fiber filament was bent into an S shape, the stimulation was continuously performed for about 1 s and repeated for 10 times at an interval of 10-15 s, and responses of the mice were observed. When the mice showed a rapid foot withdrawal or foot licking response within a stimulation time or when the von Frey fiber filament was removed, a positive response was recorded; and when no response was observed, a negative response was recorded. In 10 times of stimulation, the foot withdrawal response is expressed as a percentage of foot withdrawal frequency: (foot withdrawal number/10 tests)/100=response frequency%.

Results are shown in FIG. 8 and FIG. 9. Compared with the solvent group, the foot withdrawal frequency is remarkably reduced within 2 h after intravenous administration of the morphine single drug, indicating that the morphine single drug has an obvious analgesic effect within 2 h; the non-responsive morphine liposome has a mild analgesic effect, but no statistical differences; and the pH-responsive morphine liposome has an obvious analgesic effect within 6 h after administration. It is indicated that the pH-responsive morphine liposome drug can remarkably prolong the analgesic action time.

### 6. Side effects

After the pH-responsive morphine analgesic drug prepared in Example 1 was selected and administered through subcutaneous injection, side effects were observed, including withdrawal symptoms, effect on the heart rate, effect on blood oxygen, effect on motor coordination, effect on movement distance, and effect on defecation.

Morphine withdrawal test: Mice with neuropathic pain were continuously administered with a morphine single drug or a pH-responsive morphine liposome through subcutaneous injection at 20 mg kg⁻¹ for 6 days with 2 times a day after modeling; and 1 h after the last administration on day 6, the mice were administered with naloxone through intraperitoneal injection at 2 mg kg⁻¹, and then a total number of jumps of the mice within 30 min was observed.

Monitoring of the heart rate and blood oxygen: The heart rate and blood oxygen saturation conditions of mice were recorded by a small animal oximeter before and after subcutaneous injection of a morphine single drug or a pH-responsive morphine liposome into normal mice at 10 mg kg⁻¹ at 5, 15 and 30 min.

Rotarod test: Normal mice were placed on a rotarod instrument at a rotation speed of 4 rpm for 5 min and trained 2-3 times; after the training was completed, on the rotarod instrument with gradual acceleration at 4-45 rpm for 5 min, the movement time of the normal mice on the rotarod instrument before and after subcutaneous injection of a morphine single drug or a pH-responsive morphine liposome at 10 mg kg⁻¹ at 30 and 60 min, to reflect motor coordination of the mice.

Open field test: Normal mice were placed in an open field for adaptation for 5 min and then administered with a solvent (PBS), a morphine single drug at 10 mg kg⁻¹, or a pH-responsive morphine liposome through subcutaneous injection, and movement distance and movement trajectory conditions of the mice were recorded 15 min after the injection.

Defecation: of several fecal particles were recorded 1 h after subcutaneous injection of a solvent (PBS), a morphine single drug at 10 mg kg⁻¹, or a pH-responsive morphine liposome into normal mice.

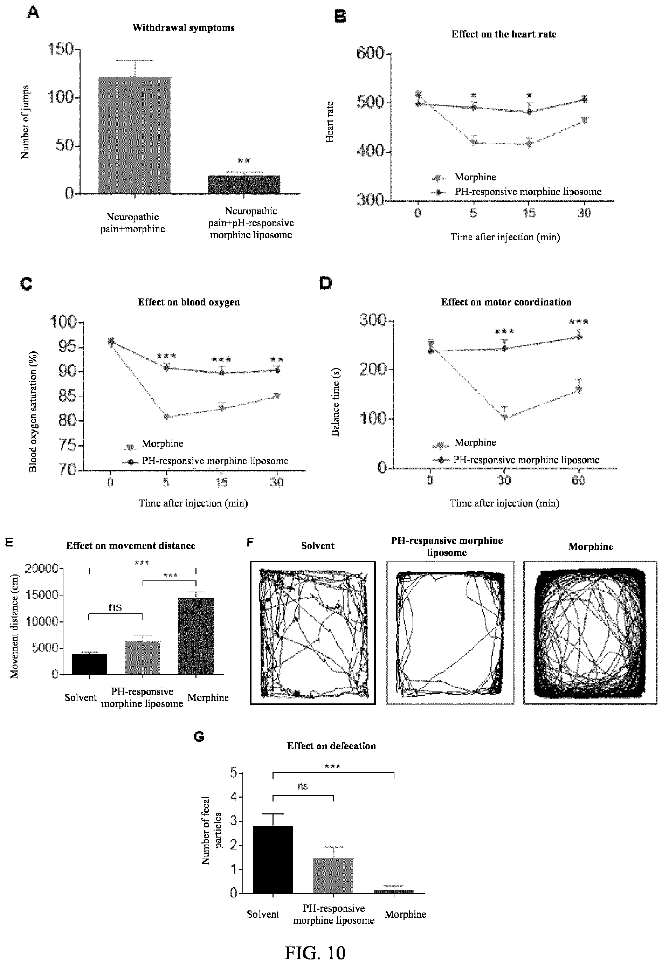

As shown in FIG. 10, (A) shows that compared with the morphine single drug, the number of jumps of the mice is remarkably reduced after subcutaneous injection of the pH-responsive morphine liposome, indicating that withdrawal symptoms are obviously relieved; (B) shows that compared with conditions before administration, the heart rate is remarkably reduced 5-30 min after subcutaneous injection of the morphine single drug, while subcutaneous injection of the pH-responsive morphine liposome has no obvious effect on the heart rate; (C) shows that compared with the morphine single drug, effect of subcutaneous injection of the pH-responsive morphine liposome on the blood oxygen saturation is obviously reduced; (D) shows that compared with the morphine single drug, subcutaneous injection of the pH-responsive morphine liposome can remarkably prolong the balance time of the mice on the rotarod instrument, indicating that the pH-responsive morphine liposome has no obvious effect on motor coordination; (E) shows that compared with the solvent group, subcutaneous injection of the morphine single drug remarkably increases the movement distance of the mice, but the pH-responsive morphine liposome has no obvious effect on the movement distance, indicating that the pH-responsive morphine liposome will not increase the movement distance of the mice; (F) shows typical movement trajectory diagrams after subcutaneous injection of the solvent, the morphine single drug and the pH-responsive morphine liposome, indicating that the movement distance is equivalent after subcutaneous injection of the solvent or the pH-responsive morphine liposome, while the movement distance is remarkably increased after subcutaneous injection of the morphine single drug; and (G) shows that compared with the solvent group, the number of fecal particles of the mice is remarkably reduced by subcutaneous injection of the morphine single drug, while the pH-responsive morphine liposome has no remarkable effect on the number of fecal particles produced by the mice, indicating that the pH-responsive morphine liposome has no obvious effect on defecation of the mice.

In conclusion, the pH-responsive morphine liposome drug can remarkably reduce the side effects of morphine on the nervous system, the circulatory system, the respiratory system, and the digestive system.

## Claims

1. Use of an injured segment of a dorsal root ganglion as a target in preparation of an analgesic drug for a peripheral nerve injury, **characterized in that** the analgesic drug is a pH-responsive analgesic drug targeting the injured segment of the dorsal root ganglion, and the pH-responsive analgesic drug is a complex formed by embedding an active analgesic ingredient in a pH-responsive carrier.

2. The use according to claim 1, **characterized in that** the pH-responsive carrier is a pH-responsive liposome and has a composition comprising phosphatidylethanolamine and oleic acid.

3. The use according to claim 1, **characterized in that** the active ingredient of the pH-responsive analgesic drug is an opioid, and the opioid is a µ opioid receptor agonist.

4. An analgesic drug targeting an injured segment of a dorsal root ganglion, **characterized in that** the analgesic drug comprises a pH-responsive carrier and an opioid embedded in the carrier.

5. The analgesic drug targeting an injured segment of a dorsal root ganglion according to claim 4, **characterized in that** the pH-responsive carrier is a pH-responsive liposome and has a composition comprising phosphatidylethanolamine and oleic acid; and a preparation method for the analgesic drug comprises the following steps:
(1) dissolving the phosphatidylethanolamine and the oleic acid in an organic solvent, and removing the organic solvent under reduced pressure to form a lipid film; and
(2) adding a buffer solution containing the opioid to undergo a hydration reaction, and performing extrusion molding through a filter membrane after complete hydration to prepare the analgesic drug.

6. The analgesic drug targeting an injured segment of a dorsal root ganglion according to claim 5, **characterized in that** a molar ratio of the phosphatidylethanolamine to the oleic acid is (1.5-4):1.

7. The analgesic drug targeting an injured segment of a dorsal root ganglion according to claim 5, **characterized in that** conditions for the hydration reaction comprise that the hydration is performed at 35-55°C for 10-20 h.

8. Use of the analgesic drug targeting an injured segment of a dorsal root ganglion according to claim 4 in preparation of drugs for treatment of neuropathic pain caused by a peripheral nerve injury.

9. The use according to claim 8, **characterized in that** the analgesic drug releases the opioid active substance in a local acidic environment of the injured segment of the dorsal root ganglion.
